(19) Europäisches Patentamt
European Patent Office
Office européen des brevets

(11) Veröffentlichungsnummer: **0 050 212**
**B1**

(12) # EUROPÄISCHE PATENTSCHRIFT

(45) Veröffentlichungstag der Patentschrift: **11.01.84**

(51) Int. Cl.³: **C 07 D 495/14**, A 61 K 31/55 // (C07D495/14, 333/00, 333/00, 223/00)

(21) Anmeldenummer: **81107343.6**

(22) Anmeldetag: **17.09.81**

(54) **5-Substituierte 9-Cyanmethylen-dithieno(3,4-b:4'.3'-e)-azepine, Verfahren zu ihrer Herstellung und diese enthaltende therapeutische Mittel.**

(30) Priorität: **08.10.80 DE 3037971**

(43) Veröffentlichungstag der Anmeldung: **28.04.82 Patentblatt 82/17**

(45) Bekanntmachung des Hinweises auf die Patenterteilung: **11.01.84 Patentblatt 84/2**

(84) Benannte Vertragsstaaten: **AT BE CH DE FR GB IT LI LU NL SE**

(56) Entgegenhaltungen:
**EP - A - 0 019 172**
**CH - A - 560 220**

(73) Patentinhaber: **BASF Aktiengesellschaft, Carl-Bosch-Strasse 38, D-6700 Ludwigshafen (DE)**

(72) Erfinder: **Steiner, Gerd, Dr., Oberer Waldweg 1, D-6719 Kirchheim (DE)**
Erfinder: **Teschendorf, Hans-Juergen, Dr., Rene-Bohn-Strasse 4, D-6700 Ludwigshafen (DE)**
Erfinder: **Kreiskott, Horst, Dr., Am Boehlig, D-6706 Wachenheim (DE)**
Erfinder: **Hofmann, Hans Peter, Dr., Untere Hart 12, D-6703 Limburgerhof (DE)**

**EP 0 050 212 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

5-substituierte 9-Cyanmethylen-dithieno[3,4-b:4'.3'-e]-azepine, Verfahren zu ihrer Herstellung und diese enthaltende therapeutische Mittel

Die Erfindung betrifft in 5-Stellung substituierte 9-Cyanmethylen-dithieno[3,4-b:4'.3'-e]azepine, Verfahren zu ihrer Herstellung und diese enthaltende therapeutische Mittel, die als Sedativa, Hypnotika, Tranquilizer, Neuroleptika oder Antiparkinsonmittel verwendet werden können.

Es ist bekannt, daß tricyclische Ringsysteme mit einer Dibenzo-Struktur zu einem zentralen heterocyclischen 7-Ring, der gegebenenfalls einen basischen Seitenrest, wie beispielsweise einen N-Methylpiperazinrest, aufweist, neuroleptische Wirkungen aufweisen können. Solche Tricyclen sind beispielsweise N-Methylpiperazin-Derivate von Dibenzo[b,e][1,4]-diazepinen (Clozapine), Dibenzo[b,f][1,4]-thiazepinen (Clotiapine), Dibenzo[b,f][1,4]-oxazepinen (Loxapine) oder Morphantridinen (Perlapine), wie beispielsweise aus der Zusammenfassung von J. Schmutz in der Arzneimittelforschung 25, 712–720 (1975) hervorgeht.

In der Patentanmeldung P 29 18 778.8 werden 6-substituierte 11-Alkylen-morphantridine mit wertvollen pharmakologischen Eigenschaften vorgeschlagen. Gegenstand dieser Anmeldung sind Dithieno[3,4-b:4'.3'-e]-Derivate, die ein unterschiedliches pharmakologisches Wirkprofil aufweisen.

Es wurde nun gefunden, daß 5-substituierte 9-Cyanmethylen-dithieno[3,4-b:4'.3'-e]-azepine der allgemeinen Formel I

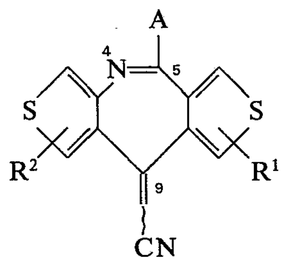

(I)

in der $R^1$ und $R^2$ Wasserstoff- oder Halogenatome, insbesondere Chlor bedeuten, und für A ein Aminorest $-NR^3R^4$ steht, in dem $R^3$ und $R^4$ zusammen mit dem sie verbindenden Stickstoffatom einen 5- bis 7gliedrigen gesättigten Ring bedeuten, der gegebenenfalls als weiteres Heteroatom ein Stickstoff- oder Sauerstoffatom enthält, wobei ein zusätzlich vorhandenes Stickstoffatom gegebenenfalls durch einen Alkylrest mit 1 bis 3 C-Atomen, einen Hydroxyalkylrest mit 2 bis 3 C-Atomen, einen Alkoxyalkylrest mit 1 bis 3 C-Atomen im Alkyl- und Alkoxyrest, einen Cycloalkyl- oder Cycloalkylmethylrest mit 3 bis 7 C-Atomen im Cycloalkylring, einen Alkinylrest mit 2 bis 5 C-Atomen und gegebenenfalls zusätzlich durch Sauerstoff in Form eines N-Oxids substituiert ist, oder A bedeutet einen Aminorest $-NHR^5$, in dem $R^5$ einen Aminoalkylrest mit 2 bis 7 C-Atomen, wobei das Aminstickstoffatom gegebenenfalls durch einen niederen Alkylrest mit 1 bis 5 C-Atomen substituiert oder Bestandteil eines 5- bis 7gliedrigen gesättigten Ringes ist, der gegebenenfalls ein Stickstoff- oder Sauerstoffatom als weiteres Heteroatom enthält, wobei ein vorhandenes Stickstoffatom durch einen niederen Alkylrest mit 1 bis 3 C-Atomen oder einen Hydroxyalkylrest mit 2 bis 3 C-Atomen substituiert ist, bedeuten, und ihre physiologisch verträglichen Säureadditionssalze wertvolle pharmakologische Eigenschaften aufweisen.

Für die Reste $R^1$ und $R^2$ seien hervorgehoben Wasserstoff und Chlor, wobei in den besonders bevorzugten Verbindungen für $R^1$ ein Wasserstoffatom und für $R^2$ ein Wasserstoff- oder Chloratom steht.

Als Aminreste $-NR^3R^4$ für A sind beispielsweise Piperazinyl-, Homopiperazinyl-, Piperidinyl- und Morpholinylreste zu nennen.

Besonders bevorzugte Reste $-NR^3R^4$ sind der 4-Methyl-piperazinyl-, 4-Methyl-4-oxy-piperazinyl-, 4-Äthyl-piperazinyl- und der N-Methyl-homopiperazinyl-Rest.

Im Aminrest $-NHR^5$ sind für $R^5$ der 2-Dimethylamino-äthyl- und der 2-Piperidin-1-yl-äthyl-Rest hervorzuheben.

Es wird darauf hingewiesen, daß die erfindungsgemäßen Verbindungen der Formel I als cis-trans-Isomere Ia und Ib vorliegen.

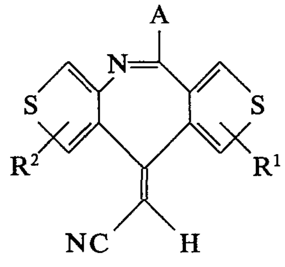

(Ia)

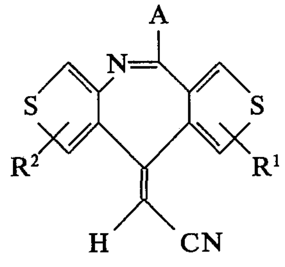

(Ib)

Die cis-trans-Isomeren können gegebenenfalls beispielsweise durch fraktionierte Kristallisation oder durch Säulenchromatographie getrennt werden.

Aufgrund der obengenannten Bedeutungen sind die folgenden Verbindungen als besonders bevorzugt und wirksam zu nennen:

cis,trans-9-Cyanmethylen-5-(4-methyl-piperazin-1-yl)dithieno[3.4-b:4'.3'-e]azepin,
cis-9-Cyanmethylen-5-(4-methyl-piperazin-1-yl)-dithieno[3.4-b:4'.3'-e]azepin,
trans-9-Cyanmethylen-5-(4-methyl-piperazin-1-yl)-dithieno[3.4-b:4'.3'-e]azepin,
cis,trans-9-Cyanmethylen-5-(4-methyl-4-oxy-piperazin-1-yl)-dithieno[3.4-b:4'.3'-e]azepin,
cis,trans-9-Cyanmethylen-5-(4-äthyl-piperazin-1-yl)-dithieno[3.4-b:4'.3']azepin,
cis-trans-9-Cyanmethylen-5-(N'-methyl-homopiperazin-1-yl)-dithieno[3.4-b:4'.3'-e]azepin,
cis-trans-3-Chlor-9-cyanmethylen-5-(4-methyl-piperazin-1-yl)-dithieno[3.4-b:4'.3'-e]azepin,
cis-trans-9-Cyanmethylen-5-(piperazin-1-yl)-dithieno[3.4-b:4'.3'-e]azepin.

Wie die Ausführungsbeispiele zeigen, kann im Einzelfall die Trennung in das cis- und trans-Isomere ohne allzu großen Aufwand vorgenommen werden.

Die erfindungsgemäßen Verbindungen der Formel I werden hergestellt, indem man eine Verbindung der Formel II

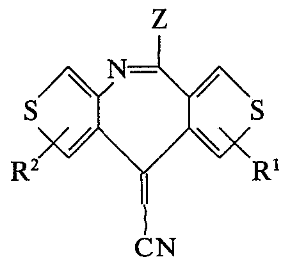

(II)

in der $R^1$ und $R^2$ die für Formel I angegebenen und bevorzugten Bedeutungen haben und Z eine nukleofuge Abgangsgruppe darstellt, mit einem Nukleophil AH, in dem A die für Formel I angegebenen Bedeutungen hat, umsetzt, gegebenenfalls in das reine cis- und trans-Isomere trennt und/oder gegebenenfalls die erhaltene Verbindung in das N-Oxid und/oder in das Säureadditionssalz einer physiologisch verträglichen Säure überführt.

Als nukleofuge Abgangsgruppe für Z kommen Halogenatome, insbesondere Brom oder Chlor, in Betracht.

Die Umsetzung erfolgt zweckmäßig in Gegenwart einer überschüssigen Menge des verwendeten Amins oder Alkohols AH, das gleichzeitig als Lösungsmittel und gegebenenfalls als säurebindendes Mittel dient. Gegebenenfalls kann in Gegenwart eines inerten Lösungsmittels, wie einem cyclischen gesättigten Äther, insbesondere Tetrahydrofuran oder Dioxan, von Benzol oder eines Benzolkohlenwasserstoffes, wie Toluol, Xylol, Mesitylen oder Decahydronaphthalin oder eines aprotischen polaren Lösungsmittels, wie Dimethylformamid, gearbeitet werden. Die Umsetzung erfolgt in der Regel bei Temperaturen von 80 bis 150°C, bevorzugt 90 bis 120°C, und ist im allgemeinen innerhalb von 3 bis 10 Stunden beendet. Gegebenenfalls ist der Ausschluß des Sauerstoffs der Luft und das Arbeiten unter Inertgas, beispielsweise unter Stickstoff, von Vorteil.

Bei den Umsetzungen wird das Nukleophil AH vorteilhafterweise in mindestens 2fachem molarem bis 20molarem Überschuß verwendet.

Die Überführung einer Verbindung der Formel I in das N-Oxid erfolgt in üblicher Weise, zweckmäßig mit wäßrigem Wasserstoffperoxid (30 Gew.-%) in äthanolischer Lösung. Die Überführung in das Säureadditionssalz einer physiologisch verträglichen Säure erfolgt ebenso in üblicher Weise.

Die Ausgangsverbindungen der Formel II werden erhalten, indem man ein 96-Cyanmethylen-dithienol[3.4-b:4'.3'-e]-4,5-dihydro-azepin-5-on der Formel III

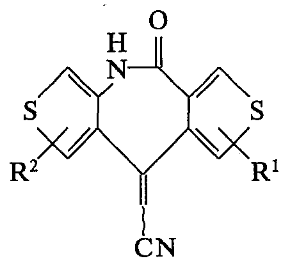

(III)

in der $R^1$ und $R^2$ die für Formel II angegebenen Bedeutungen haben, in an sich üblicher Weise mit einem überschüssigen Halogenierungsmittel, insbesondere Phosphoroxychlorid oder Phosphortribromid, gegebenenfalls in Gegenwart eines Lösungsmittels und gegebenenfalls in Gegenwart einer katalytischen Menge N,N-Dimethylanilin 3 bis 5 Stunden auf Rückflußtemperaturen oder Temperaturen bis

120°C erhitzt und das erhaltene Iminochlorid oder -bromid nach dem Abdestillieren des überschüssigen Phosphorhalogenids und Aufarbeitung in einem wäßrigen zweiphasigen System durch Extraktion mit einem chlorierten Kohlenwasserstoff, wie Chloroform oder Methylenchlorid, isoliert.

Das 9-Cyanmethylen-dithienol[3.4-b:4′.3′-e]-4,5-dihydro-azepin-5-on der Formel III, in der $R^1$, $R^2$ die für Formel I angegebenen Bedeutungen haben, wird durch Carbonyl-Olefinierung hergestellt, indem man ein Dithieno[3.4-b:4′.3′-e]-4,5-dihydro-azepin-5,9-dion der Formel IV

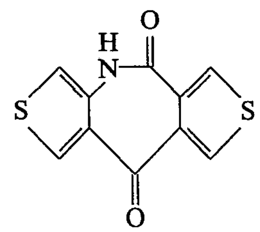

(IV)

mit einem Phosphonat der Formel Va

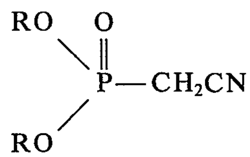

(Va)

in der R einen Alkylrest mit 1 bis 3 C-Atomen bedeutet, unter den Bedingungen der Wittig-Horner-Reaktion in einem inerten Lösungsmittel, besonders bevorzugt Dimethylformamid, in Gegenwart von einem Moläquivalent einer Base, bevorzugt Natriumalkoholat, Natriumhydrid oder Natriumamid, bei Temperaturen von 20 bis 80°C umsetzt
oder mit einem Phosphoniumsalz der Formel Vb

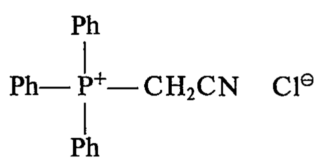

(Vb)

in der Ph für einen Phenylrest steht, unter den Bedingungen der klassischen Wittig-Horner-Reaktion in einem aprotischen organischen Lösungsmittel, insbesondere einem gesättigten aliphatischen oder gesättigten cyclischen Ether, wie Diethylether, Tetrahydrofuran oder Dioxan, oder vorzugsweise in Dimethylformamid, in Gegenwart von einem Moläquivalent einer Base, insbesondere einem Alkalialkoholat, vorzugsweise Natriummethylat oder Natriumethylat oder Natriumhydrid, Natriumamid oder einer metallorganischen Verbindung, wie Butyllithium, bei Temperaturen von 20 bis 100°C umsetzt.

Falls erforderlich, halogeniert man anschließend zur Überführung in eine Verbindung der Formel III, in der $R^1$ und/oder $R^2$ ein Chlor- oder Bromatom bedeutet, mit der zu verwendenden äquivalenten Menge Chlor oder Brom in einem inerten organischen Lösungsmittel, vorzugsweise einem Halogenkohlenwasserstoff, wie Tetrachlorkohlenstoff, Chloroform oder Methylenchlorid, bei Raumtemperatur.

Diese Halogenierung kann aber auch, wie in den Beispielen beschrieben, vorteilhaft erst auf der Endstufe, d. h. einer Verbindung der Formel I, in gleicher Weise erfolgen.

Das neue Dithieno[3.4-b:4′.3′-e]-4,5-dihydro-azepin-5,9-dion der Formel IV, in der $R^1$ und $R^2$ die für die Formel I angegebenen Bedeutungen haben, wird durch Schmidt-Ringerweiterung des 4.8-Dihydrobenzo[1.2-c:4.5-c′]-dithiophen-4,8-dions der Formel VI hergestellt.

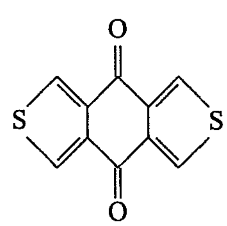

(VI)

Hierbei wird in an sich üblicher Weise das 4,8-Dihydrobenzo[1.2-c:4.5-c′]dithiophen-4,8-dion in einer Mischung aus einem chlorierten Kohlenwasserstoff, vorzugsweise Methylenchlorid, und konzentrierter Schwefelsäure (Mischungsverhältnis 1 : 2 bis 1 : 1 Volumteile) aufgenommen und bei Temperaturen von 0 bis 30°C portionsweise unter gutem Rühren 1 Moläquivalent Natriumazid eingetragen.

Die halogenierten Derivate des Dithieno[3.4-b:4′.3′-e]-4,5-dihydro-azepin-5,9-dions der Formel IV

können durch direkte Halogenierung in einem organischen Lösungsmittel, vorzugsweise einem Halogenkohlenwasserstoff, bei Temperaturen zwischen 0 und 80°C hergestellt werden.

Das Ausgangsmaterial 4,8-Dihydro-benzo[1.2-c:4.5-c']-dithiophen-4,8-dion der Formel VI ist literaturbekannt (D. W. H. MacDowell and J. C. Wisowaty, j. Org. Chem. 37, 1712 (1972)).

Neben den in den Beispielen aufgeführten Verbindungen werden folgende Verbindungen beispielhaft genannt:

cia,trans-9-Cyanmethylen-5-(4-cyclopropyl-piperazin-1-yl)-dithieno[3.4-b:4'.3'-e]azepin,
cis,trans-9-Cyanmethylen-5-(4-cyclopropylmethyl-piperazin-1-yl)-dithieno[3.4-b:4'.3'-e]azepin,
cis,trans-9-Cyanmethylen-5-(4-propin-2-yl-piperazin-1-yl)-dithieno[3.4-b:4'.3'-e]azepin,
cis,trans-3-Brom-9-cyanmethylen-5-(4-methyl-piperazin-1-yl)-dithieno[3.4-b:4'.3'-e]azepin,

Die erfindungsgemäßen Verbindungen der Formel I werden in der Regel in Form von gelblichen bis gelben Kristallen erhalten und können durch Umkristallisation aus den üblichen organischen Lösungsmitteln, bevorzugt aus einem niederen Alkohol, wie Äthanol, umkristallisiert oder durch Säulenchromatographie gereinigt werden.

Falls erforderlich, erfolgt eine Trennung in die einzelnen cis- und trans-Isomeren durch fraktionierte Kristallisation in einem chlorierten Kohlenwasserstoff, bevorzugt Methylenchlorid, einem niederen einwertigen Alkohol, bevorzugt Methanol oder Äthanol, oder einem gesättigten cycloaliphatischen Kohlenwasserstoff, bevorzugt Cyclohexan, oder durch Säulenchromatographie, insbesondere an Methylenchlorid und Methanol im Verhältnis 99 : 1 bis 85 : 15 Volumteilen.

Die freien 5-substituierten 9 Cyanmethylen-dithieno[3.4-b:4'.3'-e]azepine der Formel I können in üblicher Weise in das Säureadditionssalz einer pharmakologisch verträglichen Säure überführt werden, vorzugsweise durch Versetzen einer Lösung mit einem Äquivalent der entsprechenden Säure. Pharmazeutisch verträgliche Säuren sind beispielsweise Salzsäure, Maleinsäure und Methansulfonsäure.

Die erfindungsgemäßen Verbindungen weisen nach den pharmakologischen Ergebnissen wertvolle pharmakologische Eigenschaften auf. In Anbetracht ihrer sedativ-tranquilisierenden, muskelrelaxierenden, antimonaminergen und zentralanticholinergen Wirkung können sie als Sedativa/Hyponotika Minor- bzw. Major-Tranquilizer, Neuroleptika sowie als Antiparkinsonmittel Verwendung finden. Mehrere der genannten Wirkungsqualitäten können kombiniert bei einer erfindungsgemäßen Verbindung auftreten. In manchen Fällen kann das einzelne reine Isomere nach erfolgter Isomerentrennung eine Wirkung bevorzugt aufweisen.

Zur Wirkungsanalyse fanden folgende Methoden Verwendung:

### 1. Sedative Wirkung

4 bis 8 Gruppen von jeweils 3 weiblichen NMRI-Mäusen erhalten die Substanzen oral appliziert. Die photoelektrische Bestimmung der durch eine neue Umgebung induzierten Orientierungshypermotilität erfolgt 30 min nach der Applikation der Substanzen für eine Dauer von 30 min.

Als $ED_{50}$ wird die Dosis bestimmt, welche im Vergleich zu Placebo-behandelten Kontrolltieren eine Abnahme der Orientierungshypermotilität um 50% bewirkt.

### 2. Muskelrelaxierende Wirkung

Die Messung beruht auf der Quantifizierung des tonischen Dehnungsreflexes am Gastochemius des Kaninchen (Teschendorf et al., Arch. Pharmacol. exp. Path. 266, 462, 1970). Das Kaninchen wird in einer speziellen Apparatur fixiert, die es erlaubt, die Pfote im Sprunggelenk in definierter und reproduzierbarer Weise zu beugen. Durch die Beugung wird in der Wadenmuskulatur ein tonischer Dehnungsreflex ausgelöst. Die elektrische Aktivität des Muskels während der Kontraktion wird registriert und die Einzelimpulse gezählt. Die Dehnung (Dauer 5 s) wird in Minutenabständen wiederholt. Nach Erreichen einer konstanten Impulszahl (Kontrollwert) wird die Testsubstanz intravenös appliziert. Die Impulszahlen nach Applikation werden auf die Vorwerte bezogen.

### 3. Antimethamphetamin-Wirkung

Methamphetamin · HCl (2,5 mg/kg i.v.) ruft an Ratten regelmäßig folgende Symptome hervor: motorische Unruhe, Such- und Schnüffelbewegungen, gesträubtes Fell sowie Tremor (Janssen et al., Arzneim.-Forsch./Drug res. 13, 205, 1963; Randrup et al., Psychopharmacologia 11, 300, 1967). Die Prüfsubstanzen werden 30 min vor Methamphetamin intraperitoneal verabfolgt. Kriterium für einen Substanzeffekt ist das Ausbleiben der Schnüffelbewegungen innerhalb einer 5minutigen Beobachtungszeitraumes nach Methamphetamin-Injektion. Als mittlere Hemmdosis ($ED_{50}$) wird mittels Probitanalyse die Dosis ermittelt, die das Symptom bei der Hälfte der Tiere verhindert.

### 4. Zentral-anticholinerge Wirkung

Zur Prüfung auf zentrale anticholinerge Wirkung wird durch Pilocarpin eine cholinerge Stimulierung ausgelöst. Ratten zeigen nach rascher intravenöser Injektion von Pilocarpin · HCl (50 mg/kg) alternierende Kratzbewegungen der Hinterpfoten; weitere zentral ausgelöste Symptome sind Kieferbewegungen und Tremor. Als Zeichen peripherer Übererregung können Speicherfluß und Chromodakryorrhoe beobachtet werden (Kreiskott, Arch. exp. Path. Pharmak. 247, 317, 1964). Die Prüfsubstanzen werden 30 min vor Pilocarpin intraperitoneal verabfolgt. Kriterium für einen Substanzeffekt ist das Ausbleiben des Kratzens innerhalb 2 min nach Pilocarpin-Injektion. Für dieses Symptom wird als mittlere Hemmdosis ($ED_{50}$) mittels Probitanalyse nach Finney diejenige Dosis der Prüfsubstanz ermittelt, welche die Kratzabläufe bei der Hälfte der Tiere unterdrückt.

In diesen experimenten (vgl. Tabellè 1) wurden deutliche sedativ-hypnotische Wirkungen der erfindungsgemäßen Verbindungen nachgewiesen, die etwa gleich stark ausgeprägt sind wie bei der Referenzsubstanz Clozapin. Ebenso findet sich eine muskelrelaxierende Wirkung, welche etwas schwächer als diejenige des Clozapin ist, aber diejenige des Perlapin erreicht.

Als Parameter für eine neuroleptische Qualität kann die antimonaminerge Wirkung (Methamphetamin-Antagonismus) gewertet werden. Die Vergleichsverbindungen Clozapin und Perlapin werden in der Wirkstärke erreicht oder deutlich — bis zu 7fach — übertroffen.

Weiterin weisen die Verbindungen eine interessante zentral-anticholinerge Wirkung auf, die zum Teil deutlich stärker ausgeprägt ist als bei den Referenzsubstanzen. Diese Wirkqualität spricht zum einen für eine Verwendung der neuen Substanzen als Antiparkinsonmittel. Andererseits sind wegen dieser zentral-anticholinergen Komponente bei einer Verwendung der Verbindungen als Neuroleptika extrapyramidal-motorische Störungen nicht zu erwarten; ein solcher Zusammenhang wird für Clozapin angenommen und müßte bei den erfindungsgemäßen Verbindungen sich besonders vorteilhaft auswirken.

Tabelle 1

| Beispiel Nr. | Sedation | | Muskelrelaxation | | Methamphetamin-antagonismus | | Zentral-anticholinerge Wirkung | |
|---|---|---|---|---|---|---|---|---|
| | $ED_{50}^{+}$ | R.W.**) | $ED_{50}$ | R.W. | $ED_{50}$ | R.W. | $ED_{50}$ | R.W. |
| 1a | 7,4 | 0,64 | 0,14 | 0,33 | 5,1 | 7,25 | 2,8 | 1,1 |
| 1b cis | 5,0 | 0,94 | 0,22 | 0,21 | 5,5 | 6,72 | 0,6 | 5,2 |
| 1b trans | > 21,5 | < 0,22 | > 1,0 | < 0,046 | 77 | 0,48 | 13 | 0,24 |
| 2 | 9,6 | 0,49 | 0,08 | 0,56 | – | – | – | – |
| 6 | 10,6 | 0,44 | 0,22 | 0,21 | 42 | 0,88 | – | – |
| Clozapin | 4,7 | 1,0 | 0,046 | 1,0 | 37 | 1,0 | 3,1 | 1,0 |
| Perlapin | 2,1 | 2,24 | 0,11 | 0,42 | 31 | 1,2 | 14,0 | 0,22 |

*) mg/kg

**) R.W. = relative Wirksamkeit $\left(= \text{Quotient } \frac{ED_{50}\text{ Vergleichssubstanz}}{ED_{50}\text{ Beispiel}}\right)$

Die Erfindung betrifft dementsprechend auch ein therapeutisches Mittel, gekennzeichnet durch einen Gehalt an einer Verbindung der Formel I oder deren pharmakologisch verträgliches Säureadditionssalz als Wirkstoff neben üblichen Träger- und Verdünnungsmitteln.

Therapeutische Mittel mit üblichen Trägerstoffen oder Verdünnungsmitteln und den üblicherweise verwendeten technischen Hilfsstoffen können entsprechend der gewünschten Applikationsart und mit einer zur Anwendung geeigneten Dosiereinheit in an sich üblicher Weise hergestellt werden. Als Einzeldosen bei Menschen kommen 10 bis 100 mg in Betracht.

Die neuen Verbindungen können in den üblichen galenischen Applikationsformen, fest oder flüssig, angewendet werden, wie Tabletten, Kapseln, Pulver, Granulate, Dragees oder Lösungen. Diese werden in üblicher Weise hergestellt. Die Wirkstoffe können dabei mit den gebräuchlichen galenischen Hilfsmitteln, wie Talkum, Gummi arabicum, Saccharose, Lactose, Getreide- oder Maisstärke, Kartoffelmehl, Magnesiumstearat, Alginaten, Gummi tragacanth, Carraghenate, Polyvinylalkohol, Polyvinylpyrrolidon, wäßrigen oder nicht wäßrigen Trägern, Netzmitteln, Dispergiermitteln, Emulgatoren und/oder Konservierungsmitteln verarbeitet werden (vgl. L. G. Goodman, A. Gilman, The Pharmacological Basis of Therapeutics). Die so erhaltenen Präparate enthalten den Wirkstoff normalerweise in einer Menge von 0,001 und 99 Gew.-%.

Die bevorzugten Zubereitungen bestehen in einer Darreichungsform, die zur oralen Applikation geeignet ist. Solche Darreichungsformen sind beispielsweise Tabletten, Filmtabletten, Dragees, Kapseln, Pillen, Pulver, Lösungen, Suspensionen oder Depotformen. Es kommen auch parenterale Zubereitungen, wie Injektionslösungen, in Betracht. Weiterhin seien als Zubereitungen beispielsweise auch Suppositorien genannt.

Entsprechende Tabletten können beispielsweise durch Mischen des Wirkstoffes mit bekannten

Hilfsstoffen, beispielsweise inerten Verdünnungsmitteln, wie Dextrose, Zucker, Sorbit, Mannit, Polyvinylpyrrolidon, Calciumcarbonat, Calciumphosphat oder Milchzucker, Sprengmittel, wie Maisstärke oder Alginsäure, Bindemittel wie Stärke oder Gelatine, Gleitmittel wie Magnesiumstearat oder Talk und/ oder Mittel zur Erzielung des Depoteffektes, wie Carboxypolymethylen, Carboxymethylcellulose, Celluloseacetatphthalat oder Polyvinylacetat, erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Dabei kann auch die Drageehülle aus mehreren Schichten bestehen, wobei die oben bei den Tableten erwähnten Hilfsstoffe verwendet werden können.

Lösungen oder Suspensionen mit den erfindungsgemäßen Wirkstoffen können zusätzlich geschmacksverbessernde Mittel, wie Vanillin oder Orangenextrakt, enthalten. Sie können außerdem Suspendierstoffe, wie Natriumcarboxymethylcellulose, oder Konservierungsstoffe, wie p-Hydroxybenzoate, enthalten. Wirkstoffe enthaltende Kapseln können beispielsweise hergestellt werden, indem man den Wirkstoff mit einem inerten Träger wie Milchzucker oder Sorbit mischt und in Gelatinekapseln einkapselt. Geeignete Suppositorien lassen sich beispielsweise durch Vermischen mit dafür vorgesehenen Trägermitteln, wie Neutralfetten oder Polyethylenglykol bzw. deren Derivaten herstellen.

Die folgenden Beispiele dienen zur Erläuterung der Erfindung:

## Beispiel 1

cis- und trans-9-Cyanmethylen-5-(4-methyl-piperazin-1-yl)-dithieno[3.4-b:4'.3'-e]azepin

a) 14,0 g (54 mM) 9-Cyanmethylen-4,5-dihydro-dithieno[3.4-b:4'.3'-e]azepin-5-on (cis,trans-Isomerengemisch) wird mit 140 ml Phosphoroxychlorid und 1,5 ml N,N-Dimethylanilin versetzt und 1 Stunde unter Stickstoffatmosphäre am Rückfluß gekocht. Nach vollständigem Abdestillieren des überschüssigen Phosphoroxychlorids und Dimethylanilins am Ölpumpenvakuum verteilt man den Rückstand zwischen Methylenchlorid und Wasser, extrahiert die wäßrige Phase noch zweimal mit Methylenchlorid und wäscht die vereinigten organischen Phasen mit verdünnter HCl und Wasser gründlich nach. Trocknen und Einengen der organischen Phase liefert 14,4 g (96%) 5-Chlor-9-cyanmethylen-dithieno[3.4-b:4'.3'-e]azepin, das für die weitere Umsetzung genügend rein ist.

14,4 g (52 mM) 5-Chlor-9-cyanmethylen-dithieno[3.4-b:4'.3'-e]azepin wird mit 50 ml N-Methyl-piperazin versetzt und 2 bis 3 Stunden bei 110°C unter Stickstoff gerührt. Das dunkle homogene Reaktionsgemisch gießt man dann nach dem Abkühlen auf Eiswasser und saugt das gelbliche Rohprodukt, 9-Cyanmethylen-5-(4-methyl-piperazin-1-yl)-dithieno[3.4-b:4'.3'-e]-azepin, ab. Nach dem Trocknen im Vakuumtrockenschrank kristallisiert man das Rohprodukt aus Äthanol unter Zusatz von Aktivkohle um oder reinigt durch Säulenchromatographie (Kieselgel, Laufmittel Methylenchlorid/Methanol 95/5). Man erhält 13,6 g (85%) gelbliches 9-Cyanmethylen-5-(4-methyl-piperazin-1-yl)-dithieno[3.4-b:4'.3'-e]azepin in Form eines cis,trans-Isomerengemisches mit dem Schmp. 148–151°C.

b) Zur Trennung der cis,trans-Isomeren kristallisiert man das Isomerengemisch fraktioniert aus Äthanol um. Man isoliert als erste Fraktion 6,5 g gelbe Kristalle, die nach Ausweis des Dünnschichtchromatogramms (Kieselgel, Laufmittel Toluol/Methanol 85/15) vornehmlich aus dem unpolaren cis-Isomeren a bestehen.

Nach dem Einengen des Filtrats digeriert man den Rückstand in ca. 80 ml siedendem Cyclohexan und saugt die unlöslichen Anteile in der Hitze ab. Nach dem Nachwaschen mit wenig Cyclohexan erhält man 2,1 g gelbe Kristalle, die nach Ausweis des Dünnschichtchromatogramms (Kieselgel, Laufmittel Toluol/Methanol 85/15) vornehmlich aus dem polaren trans-Isomeren b bestehen.

Durch darauffolgende Kristallisation der angereicherten Produkte fallen aus Äthanol und Cyclohexan das cis- und trans-Isomere praktisch rein an.

Schmp.: cis-Isomeres 200–201°C, trans-Isomeres 197–198°C.

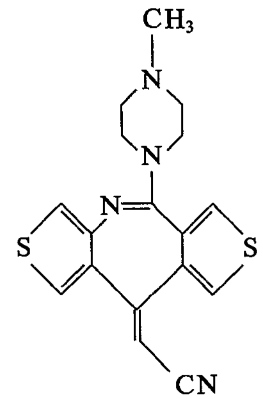 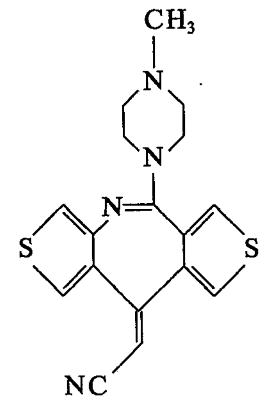

cis trans

Beispiel 2

cis,trans-3-Chlor-9-cyanmethylen-5-(4-methylpiperazin-1-yl)-dithieno[3.4-b:4'.3'-e]azepin

4,8 g (14 mM) 9-Cyanmethylen-5-(4-methyl-piperazin-1-yl)-dithieno[3.4-b:4'.3'-e]azepin (cis, trans-Isomerengemisch) werden in 160 ml Methylchlorid gelöst. Anschließend leitet man unter gutem Rühren bei 0 bis 5°C 1,0 g (14 mM) Chlor langsam ein (es fallen feinkristalline Festkörper aus). Man läßt noch 2 Stunden bei Raumtemperatur nachrühren und gießt dann den Ansatz auf Eiswasser. Nachdem man mit Natronlauge basisch gestellt hat, trennt man die organische Phase ab, extrahiert die wäßrige Phase mehrmals mit Methylenchlorid und trocknet die vereinigten organischen Phasen in üblicher Weise. Nach Einengen löst man das Rohprodukt in wenig siedendem Ethanol, fügt Aktivkohle hinzu, filtriert heiß und engt die Lösung auf ein kleines Volumen ein. Es kristallisieren in der Kälte 45%cis,trans-3-Chlor-9-cyanmethylen-5-(4-methyl-piperazin-1-yl)-dithieno[3.4-b:4'.3'-e]azepin mit Schmp. 208 bis 211°C.

Beispiel 3

cis,trans-9-Cyanmethylen-4,5-dihydro-dithieno[3.4-b:4'.3'-e]azepin-5-on

Zur Herstellung des Vorproduktes 9-Cyanmethylen-4,5-dihydro-dithieno[3.4-b:4'.3'-e]azepin-5-on führt man eine Carbonyl-Olefinierung des 4,5-Dihydro-dithieno[3.4-b:4'.3'-e]azepin-5,9-dions mit Hilfe der Wittig-Horner-Reaktion oder über die klassische Wittig-Synthese durch:

26,4 g (112 mM) 4,5-Dihydro-dithieno[3.4-b:4'.3'-e]azepin-5,9-dion werden in 300 ml Dimethylformamid unter Erwärmen gelöst und unter Stickstoff gerührt. Man läßt dann gleichzeitig 24,8 g (140 mM) Diäthyl-cyanmethyl-phosphonat und 24,6 g (140 mM) Natriumethylat (30%) gelöst in 30 ml Dimethylformamid langsam zutropfen (Farbzunahme und Temperaturerhöhung zeigen den Beginn der Wittig-Reaktion an). Nach 12stündigem Nachrühren bei Raumtemperatur gießt man das Reaktionsprodukt auf Eiswasser und saugt die ausfallenden Festkörper ab. Nach gutem Nachwaschen mit Wasser wird das Rohprodukt getrocknet und aus Äthanol umkristallisiert. Ausbeute: 26,5 g (92%) 9-Cyanmethylen-4,5-dihydro-dithieno[3.4-b:4'.3'-e]azepin-5-on, farblose Kristalle mit Schmp. 253°C (Zers.).

Klassisches Wittig-Verfahren

Man legt Triphenyl-cyanomethyl-phosphonium-chlorid in Dimethylformamid vor, läßt anschließend 1 Moläquivalent einer 30%igen Natriummethylat-Lösung zutropfen oder versetzt mit 1 Moläquivalent Natriumhydrid und fügt zuletzt 1 Moläquivalent einer Lösung von 4,5-Dihydro-dithieno[3.4-b:4'.3'-e]-azepin-5,9-dion in Dimethylformamid hinzu. Man läßt das Reaktionsgemisch 5 bis 8 Stunden bei 50 bis 80°C nachrühren, gießt anschließend auf Eiswasser und extrahiert mehrmals mit Methylenchlorid. Nach dem Trocknen und Einengen der organischen Phase kristallisiert man das Rohprodukt aus Äthanol um. Ausbeute: 61% farblose Kristalle vom Zersp. 251 bis 253°C.

## Beispiel 4

4,5-Dihydro-dithieno[3.4-b:4'.3'-e]azepin-5,9-dion

Zur Herstellung des Vorproduktes 4,5-Dihydro-dithieno[3.4-b:4'.3'-e]azepin-5,9-dion führt man eine Schmidt-Ringerweiterung durch:

30,4 g (138 mM) 4,8-Dihydro-benzo[1.2-c:4.5-c']dithiophen-4,8-dion werden in 200 ml Methylenchlorid suspendiert; unter Eiskühlung und Rühren tropft man dann 280 ml konz. Schwefelsäure hinzu. In die gut gerührte Reaktionsmischung trägt man dann bei 0°C portionsweise während 2 Stunden insgesamt 13,0 g (200 mM) Natriumazid ein. Anschließend läßt man bei Raumtemperatur 8 bis 10 Stunden nachrühren. Man gießt den Reaktionsansatz vorsichtig unter gutem Rühren in 3 l Eiswasser und saugt die nach kurzer Zeit ausfallenden Festkörper ab. Man wäscht mit reichlich Wasser bis zur neutralen Reaktion nach und trocknet das Rohprodukt im Vakuum bei 50°C.

Man isoliert 28,0 g (86%) helle Festkörper mit Schmp. 267–270°C, die für die weitere Umsetzung genügend rein sind.

## Beispiel 5

cis,trans-9-Cyanmethylen-5-(4-methyl-4-oxy-piperazin-1-yl)-dithieno[3.4-b:4'.3'-e]azepin · 2 $H_2O$

3,5 g (11 mM) cis,trans-9-Cyanmethylen-5-(4-methyl-piperazin-1-yl)-dithieno[3.4-b:4'.3'-e]azepin wird in 100 ml heißem Äthanol gelöst und mit 1,5 ml 30%igem Wasserstoffperoxid versetzt. Nach 5stündigem Rückflußkochen zerstört man das überschüssige Wasserstoffperoxid mit Hilfe eines kleinen Platinbleches, das in das Reaktionsgemisch geworfen wird, durch 2stündiges Rückflußkochen. Nach Filtrieren engt man das Reaktionsgemisch ein und reinigt das erhaltene N-Oxid durch Säulenchromatographie (Kieselgel, Laufmittel Methylenchlorid/Methanol 95/5). Man isoliert 1,8 g (50%) gelbe Kristalle mit Schmp. 137–140°C.

## Beispiel 6

cis,trans-9-Cyanmethylen-5-(4-äthyl-piperazin-1-yl)-dithieno[3.4-b:4'.3'-e]azepin

Synthese analog Beispiel 1a unter Einsatz von N-Äthylpiperazin. Nach Säulenchromatographie (Kieselgel, Laufmittel Methylenchlorid/Methanol 95/5) gelbe Kristalle mit Schmp. 118–121°C.

## Beispiel 7

cis,trans-9-Cyanmethylen-5-(N'-methyl-homopiperazin-1-yl)-dithieno[3.4-b:4'.3'-e]azepin · $H_2O$

Synthese analog Beispiel 1a unter Einsatz von N-Methylhomopiperazin. Nach Reinigung durch Säulenchromatographie (Kieselgel, Methylenchlorid/Methanol 95/5): gelbe Kristalle mit Schmp. 124 bis 126°C.

## Beispiel 8

cis,trans-9-Cyanmethylen-5-(2-piperidin-1-yl)-äthyl-amino)-dithieno[3.4-b:4'.3'-e]azepin · 1/2 $H_2O$

Synthese analog Beispiel 1a unter Einsatz von 2-Piperidin-1-yl-äthylamin. Nach Reinigung durch Säulenchromatographie (Kieselgel, Methylenchlorid/Methanol 95/5): gelbe Kristalle mit Schmp. 112 bis 114°C.

## Beispiel 9

cis,trans-9-Cyanmethylen-5-piperazin-1-yl)dithieno[3.4-b:4'.3'-e]azepin

Synthese analog Beispiel 1a unter Einsatz von 5 Moläquivalenten Piperazin und Erhöhung der Aminolyse-Temperatur auf 140°C. Nach dem Abkühlen der Reaktionsschmelze nimmt man in Methylenchlorid auf, wäscht die organische Phase dreimal mit Wasser gründlich aus und reinigt das Rohprodukt durch Säulenchromatographie (Kieselgel; Methylenchlorid/Methanol 95/5): gelbe Kristalle mit Schmp. 94–96°C.

## Beispiele für pharmazeutische Zubereitungen

Beispiele für Tabletten

1. 

| | |
|---|---|
| Ein Wirkstoff der Formel I | 10 mg |
| Lactose | 200 mg |
| Methylcellulose | 15 mg |
| Maisstärke | 50 mg |
| Talkum | 11 mg |
| Magnesiumstearat | 4 mg |

2. 

| | |
|---|---|
| Ein Wirkstoff der Formel I | 20 mg |
| Lactose | 178 mg |
| Avicel | 80 mg |
| Polywachs 6000 | 20 mg |
| Magnesiumstearat | 2 mg |

3. 

| | |
|---|---|
| Ein Wirkstoff der Formel I | 50 mg |
| Polyvinylpyrrolidon (mittl. M.G. 25 000) | 170 mg |
| Polyäthylenglykol (mittl. M.G. 4000) | 14 mg |
| Hydroxypropylmethylcellulose | 40 mg |
| Talkum | 4 mg |
| Magnesiumstearat | 2 mg |

Zu 3.

Der Wirkstoff wird mit Polyvinylpyrrolidon in 10%iger wäßriger Lösung befeuchtet, durch ein Sieb mit der lichten Maschenweite 1,0 mm getrieben und bei 50°C getrocknet. Dieses Granulat wird mit Polyethylenglykol (mittl. M.G. 4000), Hydroxypropylmethylcellulose, Talkum und Magnesiumstearat vermischt und zu Tabletten à 280 mg verpreßt.

4. Beispiel für Dragees

| | |
|---|---|
| Ein Wirkstoff der Formel I | 60 mg |
| Lactose | 90 mg |
| Maisstärke | 60 mg |
| Polyvinylpyrrolidon | 6 mg |
| Magnesiumstearat | 1 mg |

Die Mischung der Wirkstoffsubstanz mit Lactose und Maisstärke wird mit einer 8%igen wäßrigen Lösung des Polyvinylpyrrolidons durch Sieb 1,5 mm granuliert, bei 50°C getrocknet und nochmals durch Sieb 1,0 mm gerieben. Das so erhaltene Granulat wird mit Magnesiumstearat gemischt und zu Drageekernen verpreßt. Die erhaltenen Drageekerne werden in üblicher Weise mit einer Hülle überzogen, die im wesentlichen aus Zucker und Talkum bestehen.

5. Kapselformulierung

| | |
|---|---|
| Ein Wirkstoff der Formel I | 5 mg |
| Magnesiumstearat | 2,0 mg |
| Milchzucker | 19,3 mg |

6. Injektionslösung

| | |
|---|---|
| Ein Wirkstoff der Formel I | 10 mg |
| Natriumchlorid | 9 mg |
| destilliertes Wasser, q. s. auf 1,0 ml | |

**Patentansprüche für die Vertragsstaaten: BE – CH – DE – FR – GB – IT – LI – LU – NL – SE**

1. 5-substituierte 9-Cyanmethylen-dithieno[3.4-b:4'.3'-e]azepine der allgemeinen Formel I

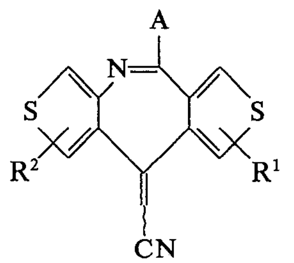

(I)

in der $R^1$ und $R^2$ Wasserstoffatome oder Halogenatome, insbesondere Chlor oder Brom bedeuten, und für A ein Aminorest $-NR^3R^4$ steht, in dem $R^3$ und $R^4$ zusammen mit dem sie verbindenden Stickstoffatom einen 5- bis 7gliedrigen gesättigten Ring bedeuten, der gegebenenfalls als weiteres Heteroatom ein Stickstoff- oder Sauerstoffatom enthält, wobei ein zusätzlich vorhandenes Stickstoffatom gegebenenfalls durch einen Alkylrest mit 1 bis 3 C-Atomen, einen Hydroxyalkylrest mit 2 bis 3 C-Atomen, einen Alkoxyalkylrest mit 1 bis 3 C-Atomen im Alkyl- und Alkoxyrest, einen Cycloalkyl- oder Cycloalkylmethylrest mit 3 bis 7 C-Atomen im Cycloalkylring, einen Alkinylrest mit 2 bis 5 C-Atomen und gegebenenfalls zusätzlich durch Sauerstoff in Form eines N-Oxids substituiert ist, oder A bedeutet einen Aminorest $-NHR^5$, in dem $R^5$ einen Aminoalkylrest mit 2 bis 7 C-Atomen, wobei das Aminstickstoffatom gegebenenfalls durch einen niederen Alkylrest mit 1 bis 5 C-Atomen substituiert oder Bestandteil eines 5- bis 7gliedrigen gesättigten Ringes ist, der gegebenenfalls ein Stickstoff- oder Sauerstoffatom als weiteres Heteroatom enthält, wobei ein vorhandenes Stickstoffatom durch einen niederen Alkylrest mit 1 bis 3 C-Atomen oder einen Hydroxyalkylrest mit 2 bis 3 C-Atomen substituiert ist, bedeuten, und ihre physiologisch verträglichen Säureadditionssalze.

2. Verbindungen der Formel I nach Anspruch 1, in denen $R^1$ und $R^2$ Wasserstoff, Chlor und Brom bedeuten und für A Piperidin, Piperazin oder Homopiperazin, am gegebenenfalls vorhandenen Ringstickstoffatom durch H, Methyl, Äthyl, $\beta$-Hydroxyäthyl, Cyclopropyl oder Propinyl substituiert und gegebenenfalls in Form des N-Oxids vorliegend, steht.

3. Verbindungen der Formel I nach Anspruch 1, in denen $R^1$ ein Wasserstoffatom und $R^2$ ein Wasserstoffatom oder ein Chloratom bedeuten und für A 4-Methyl-piperazin-1-yl, 4-Äthyl-piperazin-1-yl, 4-Methyl-4-oxy-piperazin-1-yl oder N'-Methyl-homopiperazin-1-yl steht.

4. cis,trans-9-Cyanmethylen-5-(4-methyl-piperazin-1-yl)dithieno[3.4-b:4'.3'-e]azepin.

5. cis-9-Cyanmethylen-5-(4-methyl-piperazin-1-yl)-dithieno[3.4-b:4'.3'-e]azepin.

6. trans-9-Cyanmethylen-5-(4-methyl-piperazin-1-yl)-dithieno[3.4-b:4'.3'-e]azepin.

7. cis,trans-9-Cyanmethylen-5-(4-methyl-4-oxy-piperazin-1-yl)-dithieno[3.4-b:4'.3'-e]azepin.

8. cis,trans-9-Cyanmethylen-3-chlor-5-(4-methyl-piperazin-1-yl)-dithieno[3.4-b:4'.3'-e]azepin.

9. cis,trans-9-Cyanmethylen-5-(4-äthyl-piperazin-1-yl)-dithieno[3.4-b:4'.3'-e]azepin.

10. cis,trans-9-Cyanmethylen-5-(N'-methyl-homopiperazin-1-yl)dithieno[3.4-b:4'.3'-e]azepin.

11. Verfahren zur Herstellung von Verbindungen der Formel I nach Anspruch 1, dadurch gekennzeichnet, daß man eine Verbindung der Formel II

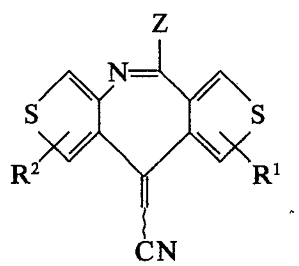

(II)

in der $R^1$ und $R^2$ die für Formel I angegebenen Bedeutungen haben und Z eine nukleofuge Abgangsgruppe darstellt, mit einem Nukleophil AH, in dem A die für Formel I angegebenen Bedeutungen hat, umsetzt und gegebenenfalls die erhaltene Verbindung in das N-Oxid und/oder in das Säureadditionssalz einer physiologisch verträglichen Säure überführt.

12. Therapeutisches Mittel, gekennzeichnet durch einen Gehalt an einer Verbindung der Formel I nach Anspruch 1 oder deren pharmakologisch verträgliches Säureadditionssalz als Wirkstoff neben üblichen Träger- und Verdünnungsmitteln.

13. Eine Verbindung der Formel I nach Anspruch 1 zur Verwendung als Sedativum, Hypnotikum, Tranquilizer und Antiparkinsonmittel.

**Patentansprüche für den Vertragsstaat: AT**

1. Verfahren zur Herstellung von 5-substituierten 9-Cyanmethylen-dithieno[3.4-b:4'.3'-e]-azepinen der allgemeinen Formel I

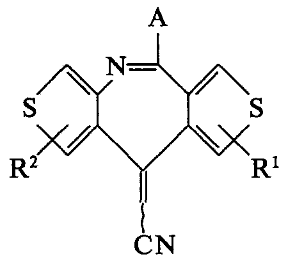

(I)

in der $R^1$ und $R^2$ Wasserstoffatome oder Halogenatome, insbesondere Chlor oder Brom bedeuten, und für A ein Aminorest $-NR^3R^4$ steht, in dem $R^3$ und $R^4$ zusammen mit dem sie verbindenden Stickstoffatom einen 5- bis 7gliedrigen gesättigten Ring bedeuten, der gegebenenfalls als weiteres Heteroatom ein Stickstoff- oder Sauerstoffatom enthält, wobei ein zusätzlich vorhandenes Stickstoffatom gegebenenfalls durch einen Alkylrest mit 1 bis 3 C-Atomen, einen Hydroxyalkylrest mit 2 bis 3 C-Atomen, einen Alkoxyalkylrest mit 1 bis 3 C-Atomen im Alkyl- und Alkoxyrest, einen Cycloalkyl- oder Cycloalkylmethylrest mit 3 bis 7 C-Atomen im Cycloalkylring, einen Alkinylrest mit 2 bis 5 C-Atomen und gegebenenfalls zusätzlich durch Sauerstoff in Form eines N-Oxids substituiert ist, oder A bedeutet einen Aminorest $-NHR^5$, in dem $R^5$ einen Aminoalkylrest mit 2 bis 7 C-Atomen, wobei das Aminstickstoffatom gegebenenfalls durch einen niederen Alkylrest mit 1 bis 5 C-Atomen substituiert oder Bestandteil eines 5- bis 7gliedrigen gesättigten Ringes ist, der gegebenenfalls ein Stickstoff- oder Sauerstoffatom als weiteres Heteroatom enthält, wobei ein vorhandenes Stickstoffatom durch einen niederen Alkylrest mit 1 bis 3 C-Atomen oder einen Hydroxyalkylrest mit 2 bis 3 C-Atomen substituiert ist, bedeuten, und ihren physiologisch verträglichen Säureadditionssalzen, dadurch gekennzeichnet, daß man eine Verbindung der Formel II

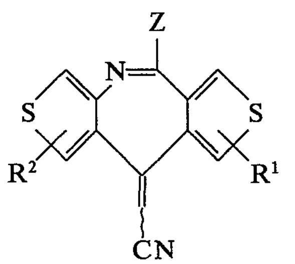

(II)

in der $R^1$ und $R^2$ die für Formel I angegebenen Bedeutungen haben und Z eine nukleofuge Abgangsgruppe darstellt, mit einem Nukleophil AH, in dem A die für Formel I angegebenen Bedeutungen hat, umsetzt und gegebenenfalls die erhaltene Verbindung in das N-Oxid und/oder in das Säureadditionssalz einer physiologisch verträglichen Säure überführt.

2. Verfahren zur Herstellung von Verbindungen der Formel I nach Anspruch 1, in denen $R^1$ und $R^2$ Wasserstoff, Chlor und Brom bedeuten und für A Piperidin, Piperazin oder Homopiperazin, am gegebenenfalls vorhandenen Ringstickstoffatom durch H, Methyl, Äthyl, $\beta$-Hydroxyäthyl, Cyclopropyl oder Propinyl substituiert und gegebenenfalls in Form des N-Oxids vorliegend, steht.

3. Verfahren zur Herstellung von Verbindungen der Formel I nach Anspruch 1, in denen $R^1$ ein Wasserstoffatom und $R^2$ ein Wasserstoffatom oder ein Chloratom bedeuten und für A 4-Methyl-piperazin-1-yl, 4-Äthyl-piperazin-1-yl, 4-Methyl-4-oxy-piperazin-1-yl oder N'-Methyl-homopiperazin-1-yl steht.

**Claims for the Contracting States: BE, CH, DE, FR, GB, IT, LI, LU, NL, SE**

1. A 5-substituted 9-cyanomethylene-dithieno-[3,4-b:4',3'-e]azepine of the general formula I

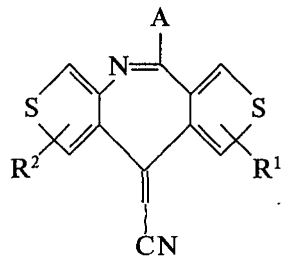

(I)

where $R^1$ and $R^2$ are hydrogen or halogen, especially chlorine or bromine, and A is $-NR^3R^4$, where $R^3$ and $R^4$, together with the nitrogen atom linking them, are a 5-membered to 7-membered saturated ring, which may contain nitrogen or oxygen as a further hetero-atom, an additional nitrogen present being unsubstituted or substituted by alkyl of 1 to 3 carbon atoms, hydroxyalkyl of 2 or 3 carbon atoms, alkoxyalkyl, where alkyl and alkoxy are of 1 to 3 carbon atoms, cycloalkyl or cycloalkylmethyl, where cycloalkyl is of 3 to 7 carbon atoms or alkynyl of 2 to 5 carbon atoms, and may additionally be substituted by oxygen in the form of an N-oxide, or A is $-NHR^5$, where $R^5$ is aminoalkyl of 2 to 7 carbon atoms, the amine nitrogen being unsubstituted or substituted by lower alkyl of 1 to 5 carbon atoms or being a constituent of a 5-membered to 7-membered saturated ring, which may contain nitrogen or oxygen as a further hetero-atom, a nitrogen present being substituted by lower alkyl of 1 to 3 carbon atoms or hydroxyalkyl of 2 or 3 carbon atoms, and its physiologically tolerated acid addition salts.

2. A compound of the formula I as claimed in claim 1, where $R^1$ and $R^2$ are hydrogen, chlorine or bromine and A is piperidine, piperazine or homopiperazine, in which any ring nitrogen atom present is substituted by H, methyl, ethyl, β-hydroxyethyl, cyclopropyl or propynyl and may be in the form of the N-oxide.

3.. A compound of the formula I as claimed in claim 1, where $R^1$ is hydrogen, $R^2$ is hydrogen or chlorine and A is 4-methyl-piperazin-1-yl, 4-ethyl-piperazin-1-yl, 4-methyl-4-oxy-piperazin-1-yl or N'-methyl-homopiperazin-1-yl.

4. Cis/trans-9-cyanomethylene-5-(4-methyl-piperazin-1-yl)-dithieno[3,4-b:4',3'-e]azepine.

5. Cis-9-cyanomethylene-5-(4-methyl-piperazin-1-yl)-dithieno[3,4-b:4',3'-e]azepine.

6. Trans-9-cyanomethylene-5-(4-methyl-piperazin-1-yl)-dithieno[3,4-b:4',3'-e]azepine.

7. Cis-trans-9-cyanomethylene-5-(4-methyl-4-oxy-piperazin-1-yl)-dithieno[3,4-b:4',3'-e]azepine.

8. Cis/trans-9-cyanomethylene-3-chloro-5-(4-methyl-piperazin-1-yl)-dithieno[3,4-b:4',3'-e]-azepine.

9. Cis/trans-9-cyanomethylene-5-(4-ethyl-piperazin-1-yl)-dithieno[3,4-b:4',3'-e]azepine.

10. Cis/trans-9-cyanomethylene-5-(N'-methyl-homopiperazin-1-yl)-dithieno[3,4-b:4',3'-e]azepine.

11. A process for the preparation of a compound of the formula I as claimed in claim 1, wherein a compound of the formula II

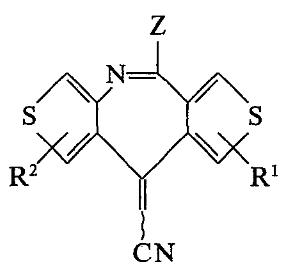

(II)

where $R^1$ and $R^2$ have the meanings given in the case of formula I and Z is a nucleofugic leaving group, is reacted with a nucleophile AH, where A has the meanings given in the case of formula I, and, if desired, the resulting compound is converted into the N-oxide and/or into the acid addition salt of a physiologically tolerated acid.

12. A therapeutic agent, which contains, in addition to conventional carriers and diluents, a compound of the formula I as claimed in claim 1, or a pharmacologically tolerated acid addition salt thereof, as the active compound.

13. A compound of the formula I as claimed in claim 1, for use as a sedative, hypnotic drug, tranquillizer or anti-Parkinson drug.

**Claims for the Contracting State: AT**

1. A process for the preparation of a 5-substituted 9-cyanomethylene-dithieno-[3,4-b:4',3'-e]azepine of the general formula I

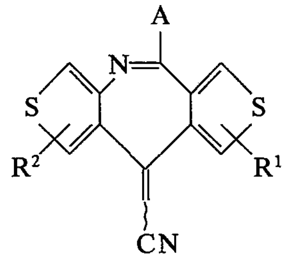

(I)

where $R^1$ and $R^2$ are hydrogen or halogen, especially chlorine or bromine, and A is $-NR^3R^4$, where $R^3$ and $R^4$, together with the nitrogen atom linking them, are a 5-membered to 7-membered saturated ring, which may contain nitrogen or oxygen as a further hetero-atom, an additional nitrogen present being unsubstituted or substituted by alkyl of 1 to 3 carbon atoms, hydroxyalkyl of 2 or 3 carbon atoms, alkoxyalkyl, where alkyl and alkoxy are of 1 to 3 carbon atoms, cycloalkyl or cycloalkylmethyl, where cycloalkyl is of 3 to 7 carbon atoms or alkynyl of 2 to 5 carbon atoms, and may additionally be substituted by oxygen in the form of an N-oxide, or A is $-NHR^5$, where $R^5$ is aminoalkyl of 2 to 7 carbon atoms, the amine nitrogen being unsubstituted or substituted by lower alkyl of 1 to 5 carbon atoms or being a constituent of a 5-membered to 7-membered saturated ring, which may contain nitrogen or oxygen as a further hetero-atom, a nitrogen present being substituted by lower alkyl of 1 to 3 carbon atoms or hydroxyalkyl of 2 or 3 carbon atoms, and its physiologically tolerated acid addition salts, wherein a compound of the formula II

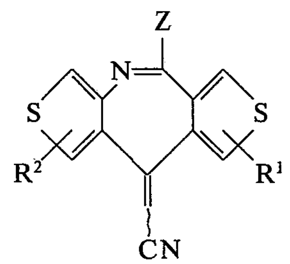

(II)

where $R^1$ and $R^2$ have the meanings given in the case of formula I and Z is a nucleofugic leaving group, is reacted with a nucleophile AH, where A has the meanings given in the case of formula I, and, if desired, the resulting compound is converted into the N-oxide and/or into the acid addition salt of a physiologically tolerated acid.

2. A process for the preparation of a compound of the formula I as claimed in claim 1, where $R^1$ and $R^2$ are hydrogen, chlorine or bromine and A is piperidine, piperazine or homopiperazine, in which any ring nitrogen atom present is substituted by H, methyl, ethyl, $\beta$-hydroxyethyl, cyclopropyl or propynyl and may be in the form of the N-oxide.

3. A process for the preparation of a compound of the formula I as claimed in claim 1, where $R^1$ is hydrogen, $R^2$ is hydrogen or chlorine and A is 4-methyl-piperazin-1-yl, 4-ethyl-piperazin-1-yl, 4-methyl-4-oxy-piperazin-1-yl or N'-methyl-homopiperazin-1-yl.

**Revendications pour les Etats contractants: BE, CH, DE, FR, GB, IT, LI, LU, NL, SE**

1. 9-cyanométhylen-dithiéno[3.4-b:4'.3'-e]azépine substuée en 5, de formule générale I

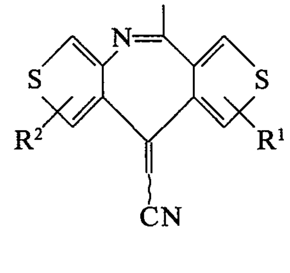

(I)

dans laquelle $R^1$ et $R^2$ représentent des atomes d'hydrogène ou d'halogène, en particulier chlore ou brome, et A est mis pour un reste amino $-NR^3R^4$, dans lequel $R^3$ et $R^4$, ensemble avec l'atome d'azote qui les relie, représentent un cycle saturé à 5 à 7 chaînons, qui contient éventuellement, comme autre hétéroatome, un atome d'azote ou d'oxygène, un atome d'atome présent en plus étant éventuellement substitué par un reste alkyle ayant 1 à 3 atomes C, un reste hydroxyalkyle ayant 2 à 3 atomes C, un reste alcoxyalkyle ayant 1 à 3 atomes C dans le reste alkyle et alcoxy, un reste cycloalkyle ou cycloalkylméthyle ayant 3 à 7 atomes C dans le cycle cycloalkyle, un reste alcinyle ayant 2 à 5 atomes C et éventuellement substitué en plus par l'oxygène sous forme d'un N-oxyde, ou bien A représente un reste amino $-NHR^5$,dans lequel $R^5$ est un reste aminoalkyle ayant 2 à 7 atomes C, l'atome d'azote d'amine étant éventuellement substitué par un reste alkyle inférieur ayant 1 à 5 atomes C ou étant partie d'un cycle saturé à 5 à 7 chaînons et qui contient éventuellement un atome d'azote ou d'oxygène comme autre hétéroatome, un atome d'azote présent étant substitué par un reste alkyle inférieur ayant 1 à 3 atomes C ou un reste hydroxyalkyle ayant 2 à 3 atomes C,
et leurs sels d'addition d'acide tolérables physiologiquement.

2. Composés de formule I selon la revendication 1, dans lesquels $R^1$ et $R^2$ représentent hydrogène, chlore et brome, et A est mis pour pipéridine, pipérazine ou homopipérazine, substitué sur l'atome d'azote du cycle, éventuellement présent, par H, méthyle, éthyle, $\beta$-hydroxyéthyle, cyclopropyle ou propinyle et existant éventellement sous forme de N-oxyde.

3. Composés de formule I selon la revendication 1, dans lequel $R^1$ représente un atome d'hydrogène et $R^2$ un atome d'hydrogène ou un atome de chlore et A est mis pour 4-méthyl-pipérazin-1-yle, 4-éthyl-pipérazin-1-yle, 4-méthyl-4-oxypipérazin-1-yle ou N'-méthyl-homopipérazin-1-yle.

4. cis,trans-9-cyanométhylen-5-(4-méthyl-pipérazin-1-yl)-dithiéno[3.4-b:4'.3'-e]azépine.

5. cis-9-cyanométhylen-5-(4-méthyl-pipérazin-1-yl)-dithieno[3.4-b:4'.3'-e]azépine.

6. trans-9-cyanométhylen-5-(4-méthyl-piperazin-1-yl)-dithieno[3.4-b:4'.3'-e]azépine.

7. cis,trans-9-cyanométhylen-5-(4-méthyl-4-oxy-pipérazin-1-yl)-dithieno[3.4-b:4'.3'-e]azépine.

8. cis,trans-9-cyanométhylen-3-chloro-5-(4-méthyl-pipérazin-1-yl)-dithieno[3.4-b:4'.3'-e]-azépine.

9. cis,trans-9-cyanométhylen-5-(4-éthyl-pipérazin-1-yl)-dithiéno[3.4-b:4'.3'-e]azépine.

10. cis,trans-9-cyanométhylen-5-(N'-méthyl-homopiperazin-1-yl)-dithiéno[3.4-b:4'.3'-e]azépine.

11. Procédé de préparation de composés de formule I selon la revendication 1, caractérisé par le fait qu'on fait réagir un composé de formule II

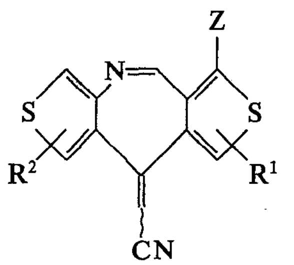

(II)

dans laquelle $R^1$ et $R^2$ ont les significations données pour la formule I et Z représente un groupe de départ nucléofuge, avec un nucléophile AH, dans lequel A a la signification donnée pour la formule I et on transforme éventuellement le composé obtenu en le N-oxyde et/ou en le sel d'addition d'acide d'un acide compatible physiologiquement.

12. Agent thérapeutique caractérisé par le fait qu'il contient comme principe actif un composé de formule I selon la revendication 1 ou son sel d'addition d'acide acceptable du point de vue pharmacologique à côté de véhicules et diluants usuels.

13. Un composé de formule I selon la revendication 1, pour l'utilisation comme sédatif, hypnoptique, tranquillisant et agent antiparkinson.

**Revendications pour l'Etat contractant: AT**

1. Procédé de préparation de 9-cyanométhylen-dithiéno[3.4-b:4'.3'-e]-azépine substitué en 5, de formule générale I

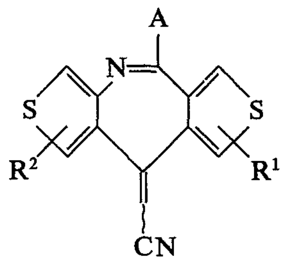

(I)

dans laquelle $R^1$ et $R^2$ représentent des atomes d'hydrogène ou d'halogène, en particulier chlore ou brome, et A est mis pour un reste amino $-NR^3R^4$, dans lequel $R^3$ et $R^4$, ensemble avec l'atome d'azote qui les relie, représentent un cycle saturé à 5 à 7 chaînons, qui contient éventuellement, comme autre hétéroatome, un atome d'azote ou d'oxygène, un atome d'atome présent en plus étant éventuellement substitué par un reste alkyle ayant 1 à 3 atomes C, un reste hydroxyalkyle ayant 2 à 3 atomes C, un reste alcoxyalkyle ayant 1 à 3 atomes C dans le reste alkyle et alcoxy, un reste cycloalkyle ou cycloalkylméthyle ayant 3 à 7 atomes C dans le cycle cycloalkyle, un reste alcinyle ayant 2 à 5 atomes C et éventuellement substitué en plus par l'oxygène sous forme d'un N-oxyde, ou bien A représente un reste amino $-NHR^5$, dans lequel $R^5$ est un reste aminoalkyle ayant 2 à 7 atomes C, l'atome d'azote d'amine étant éventuellement substitué par un reste alkyle inférieur ayant 1 à 5 atomes C ou étant partie d'un cycle saturé à 5 à 7 chaînons et qui contient éventuellement un atome d'azote ou d'oxygène comme autre hétéroatome, un atome d'azote présent étant substitué par un reste alkyle inférieur ayant 1 à 3 atomes C ou un reste hydroxyalkyle ayant 2 à 3 atomes C,
et leurs sels d'addition d'acide tolérables physiologiquement, caractérisé par le fait qu'on fait réagir un composé de formule II

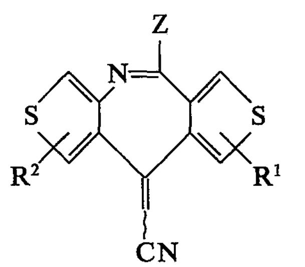

(II)

dans laquelle $R^1$ et $R^2$ ont les significations données pour la formule I et Z représente un groupe de départ nucléofuge, avec un nucléophile AH, dans lequel A a la signification donnée pour la formule I et on transforme éventuellement le composé obtenu en le N-oxyde et/ou en le sel d'addition d'acide d'un acide compatible physiologiquement.

2. Procédé de préparation de composés de formule I selon la revendication 1, dans lesquels $R^1$ et $R^2$ représentent hydrogène, chlore et brome, et A est mis pour pipéridine, pipérazine ou homopipérazine, substitué sur l'atome d'azote du cycle, éventuellement présent, par H, méthyle, éthyle, β-hydroxyéthyle, cyclopropyle ou propinyle et existant éventuellement sous forme de N-oxyde.

3. Procédé de préparation de composés de formule I selon la revendication 1, dans lequel $R^1$ représente un atome d'hydrogène et $R^2$ un atome d'hydrogène ou un atome de chlore et A est mis pour 4-méthyl-pipérazin-1-yle, 4-éthyl-pipérazin-1-yle, 4-méthyl-4-oxypipérazin-1-yle ou N'-méthyl-homopipérazin-1-yle.